(19) Europäisches Patentamt
European Patent Office
Office européen des brevets

(11) EP 1 647 309 A1

(12) EUROPÄISCHE PATENTANMELDUNG

(43) Veröffentlichungstag:
**19.04.2006 Patentblatt 2006/16**

(51) Int Cl.:
***A61Q 17/04** (2006.01)* ***A61K 8/49** (2006.01)*

(21) Anmeldenummer: **05011451.1**

(22) Anmeldetag: **18.05.2000**

(84) Benannte Vertragsstaaten:
**AT BE CH CY DE DK ES FI FR GB GR IE IT LI LU MC NL PT SE**

(30) Priorität: **22.05.1999 DE 19923672**

(62) Dokumentnummer(n) der früheren Anmeldung(en) nach Art. 76 EPÜ:
**00110560.0 / 1 055 421**

(71) Anmelder: **Beiersdorf AG**
**20253 Hamburg (DE)**

(72) Erfinder:
- **Gers-Barlag, Heinrich, Dr.**
  **25495 Kummerfeld (DE)**
- **Müller, Anja**
  **26789 Leer (DE)**

Bemerkungen:
Diese Anmeldung ist am 27-05-2005 als Teilanmeldung zu der unter INID-Kode 62 erwähnten Anmeldung eingereicht worden.

(54) **Kosmetische oder dermatologische Lichtschutzzubereitungen mit einem Gehalt an Dioctylbutamidotriazon und einem oder mehreren Filmbildnern und einem oder mehreren Polyolen**

(57) Kosmetische oder dermatologische Lichtschutzzubereitungen mit einem Gehalt an

(a) Dioctylbutamidotriazon,
(b) einem oder mehreren Filmbildnem sowie
(c) einem oder mehreren Polyolen

sowie die Verwendung von

(a) Dioctylbutamidotriazon,
(b) einem oder mehreren Filmbildnem sowie
(c) einem oder mehreren Polyolen

zur Herstellung kosmetisch oder dermatologisch akzeptabler Zubereitungen welche einen lichtschutzwirksamen Film auf der Haut hinterlassen.

EP 1 647 309 A1

**Beschreibung**

Die vorliegende Erfindung betrifft Lichtschutzformulierungen, insbesondere kosmetische und dermatologische Lichtschutzmittel.

Die schädigende Wirkung des ultravioletten Teils der Sonnenstrahlung auf die Haut ist allgemein bekannt. Während Strahlen mit einer Wellenlänge, die kleiner als 290 nm ist (der sogenannte UVC-Bereich), von der Ozonschicht in der Erdatmosphäre absorbiert werden, verursachen Strahlen im Bereich zwischen 290 nm und 320 nm, dem sogenannten UVB-Bereich, ein Erythem, einen einfachen Sonnenbrand oder sogar mehr oder weniger starke Verbrennungen.

Als ein Maximum der Erythemwirksamkeit des Sonnenlichtes wird der engere Bereich um 308 nm angegeben.

Zum Schutz gegen UVB-Strahlung sind zahlreiche Verbindungen bekannt, bei denen es sich um Derivate des 3-Benzylidencamphers, der 4-Aminobenzoesäure, der Zimtsäure, der Salicylsäure, des Benzophenons sowie auch des 2-Phenylbenzimidazols handelt.

Auch für den Bereich zwischen etwa 320 nm und etwa 400 nm, den sogenannten UVA-Bereich, ist es wichtig, Filtersubsubstanzen zur Verfügung zu haben, da auch dessen Strahlen Schäden hervorrufen können. Man hat lange Zeit fälschlicherweise angenommen, daß die langwellige UV-A-Strahlung mit einer Wellenlänge zwischen 320 nm und 400 nm nur eine vemachlässigbare biologische Wirkung aufweist und daß dementsprechend die UV-B-Strahlen für die meisten Lichtschäden an der menschlichen Haut verantwortlich seien. Inzwischen ist allerdings durch zahlreiche Studien belegt, daß UV-A-Strahlung im Hinblick auf die Auslösung photodynamischer, speziell phototoxischer Reaktionen und chronischer Veränderungen der Haut weitaus gefährlicher als UV-B-Strahlung ist. Auch kann der schädigende Einfluß der UV-B-Strahlung durch UV-A-Strahlung noch verstärkt werden.

So ist es u.a. erwiesen, daß selbst die UV-A-Strahlung unter ganz normalen Alltagsbedingungen ausreicht, um innerhalb kurzer Zeit die Collagen- und Elastinfasern zu schädigen, die für die Struktur und Festigkeit der Haut von wesentlicher Bedeutung sind. Hierdurch kommt es zu chronischen lichtbedingten Hautveränderungen - die Haut "altert" vorzeitig. Zum klinischen Erscheinungsbild der durch Licht gealterten Haut gehören beispielsweise Falten und Fältchen sowie ein unregelmäßiges, zerfurchtes Relief. Ferner können die von lichtbedingter Hautalterung betroffenen Partien eine unregelmäßige Pigmentierung aufweisen. Auch die Bildung von braunen Flecken, Keratosen und sogar Karzinomen bzw. malignen Melanomen ist möglich. Eine durch die alltägliche UV-Belastung vorzeitig gealterte Haut zeichnet sich außerdem durch eine geringere Aktivität der Langerhanszellen und eine leichte, chronische Entzündung aus.

Etwa 90 % der auf die Erde gelangenden ultravioletten Strahlung besteht aus UV-A-Strahlen. Während die UV-B-Strahlung in Abhängigkeit von zahlreichen Faktoren stark variiert (z.B. Jahres- und Tageszeit oder Breitengrad), bleibt die UV-A-Strahlung unabhängig von jahres- und tageszeitlichen oder geographischen Faktoren Tag für Tag relativ konstant. Gleichzeitig dringt der überwiegende Teil der UV-A-Strahlung in die lebende Epidermis ein, während etwa 70 % der UV-B-Strahlen von der Homschicht zurückgehalten werden.

Vorbeugender Schutz gegen UV-A-Strahlen, beispielsweise durch Auftrag von Lichtschutzfiltersubstanzen in Form einer kosmetischen oder dermatologischen Formulierung auf die Haut, ist daher von grundsätzlicher Wichtigkeit.

Im allgemeinen ist das Lichtabsorptionsverhalten von Lichtschutzfiltersubstanzen sehr gut bekannt und dokumentiert, zumal in den meisten Industrieländern Positivlisten für den Einsatz solcher Substanzen existieren, welche recht strenge Maßstäbe an die Dokumentation anlegen. Für die Dosierung der Substanzen in den fertigen Formulierungen können die Extinktionswerte allenfalls eine Orientierungshilfe bieten, denn durch Wechselwirkungen mit Inhaltsstoffen der Haut oder der Hautoberfläche selbst können Unwägbarkeiten auftreten. Ferner ist in der Regel schwierig vorab abzuschätzen, wie gleichmäßig und in welcher Schichtdicke die Filtersubstanz in und auf der Hornschicht der Haut verteilt ist.

Zur Prüfung der UV-A-Schutzleistung wird üblicherweise die IPD-Methode verwendet (IPD ≡ immediate pigment darkening). Hierbei wird - ähnlich der Bestimmung des Lichtschutzfaktors - ein Wert ermittelt, der angibt, um wieviel länger die mit dem Lichtschutzmittel geschützte Haut mit UV-A-Strahlung bestrahlt werden kann, bis die gleiche Pigmentierung auftritt wie bei der ungeschützten Haut.

Eine andere, europaweit etablierte Prüfungsmethode ist der Australische Standard AS/NZS 2604:1997. Dabei wird die Absorption der Zubereitung im UV-A-Bereich gemessen. Um den Standard zu erfüllen, muß die Zubereitung mindestens 90 % der UV-A-Strahlung im Bereich 320 -360 nm absorbieren.

Die Einsatzkonzentration bekannter Lichtschutzfiltersubstanzen, die insbesondere auch im UV-A-Bereich eine hohe Filterwirkung zeigen, ist häufig gerade in Kombination mit anderen als Festkörper vorliegenden Substanzen begrenzt. Es bereitet daher gewisse formulierungstechnische Schwierigkeiten, höhere Lichtschutzfaktoren bzw. UV-A-Schutzleistung zu erzielen.

Da Lichtschutzfiltersubstanzen in der Regel kostspielig sind und da manche Lichtschutzfiltersubstanzen zudem schwierig in höheren Konzentrationen in kosmetische oder dermatologische Zubereitungen einzuarbeiten sind, war es eine Aufgabe der Erfindung, auf einfache und preiswerte Weise zu Zubereitungen zu gelangen, welche bei ungewöhnlich niedrigen Konzentrationen an herkömmlichen UV-A-Lichtschutzfiltersubstanzen dennoch eine akzeptable oder sogar hohe UV-A-Schutzleistung erreichen.

Die UV-Strahlung kann aber auch zu photochemischen Reaktionen führen, wobei dann die photochemischen Reaktionsprodukte in den Hautmetabolismus eingreifen. Vorwiegend handelt es sich bei solchen photochemischen Reaktionsprodukten um radikalische Verbindungen, beispielsweise Hydroxyradikale. Auch undefinierte radikalische Photoprodukte, welche in der Haut selbst entstehen, können aufgrund ihrer hohen Reaktivität unkontrollierte Folgereaktionen an den Tag legen. Aber auch Singulettsauerstoff, ein nichtradikalischer angeregter Zustand des Sauerstoffmoleküls kann bei UV-Bestrahlung auftreten, ebenso kurzlebige Epoxide und viele Andere. Singulettsauerstoff beispielsweise zeichnet sich gegenüber dem normalerweise vorliegenden Triplettsauerstoff (radikalischer Grundzustand) durch gesteigerte Reaktivität aus. Allerdings existieren auch angeregte, reaktive (radikalische) Triplettzustände des Sauerstoffmoleküls.

Um diesen Reaktionen vorzubeugen, können den kosmetischen bzw. dermatologischen Formulierungen zusätzlich Antioxidantien und/oder Radikalfänger einverleibt werden.

Bekannte und vorteilhafte Lichtschutzfiltersubstanzen sind Dibenzoylmethanderivate, beispielsweise 5-Isopropyldibenzoylmethan (CAS-Nr. 63250-25-9), sowie das 4-(tert.-Butyl)-4'-methoxydibenzoylmethan (CAS-Nr. 70356-09-1). Gerade in Kombination mit anderen als Festkörper vorliegenden Substanzen ist ihre Einsatzkonzentration jedoch begrenzt. Es bereitet daher gewisse formulierungstechnische Schwierigkeiten, höhere Lichtschutzfaktoren zu erzielen.

Eine weitere vorteilhafte Lichtschutzfiltersubstanz ist der 4-Methylbenzylidencampher. Dieser ist eine äußerst vorteilhafte, unter Normalbedingungen als Festkörper vorliegende Lichtschutzfiltersubstanz und zeichnet sich an sich durch gute UV-Filtereigenschaften aus. Gerade in Kombination mit anderen als Festkörper vorliegenden Substanzen ist jedoch auch ihre Einsatzkonzentration jedoch begrenzt. Es bereitet daher auch hier gewisse formulierungstechnische Schwierigkeiten, höhere Lichtschutzfaktoren zu erzielen.

Ein weiterer vorteilhafter UV-Filter ist das 2,4-Bis-{[4-(2-Ethyl-hexyloxy)-2-hydroxy)-phenyl}-6-(4-methoxyphenyl)-1,3,5-triazin, welches durch folgende Struktur gekennzeichnet ist:

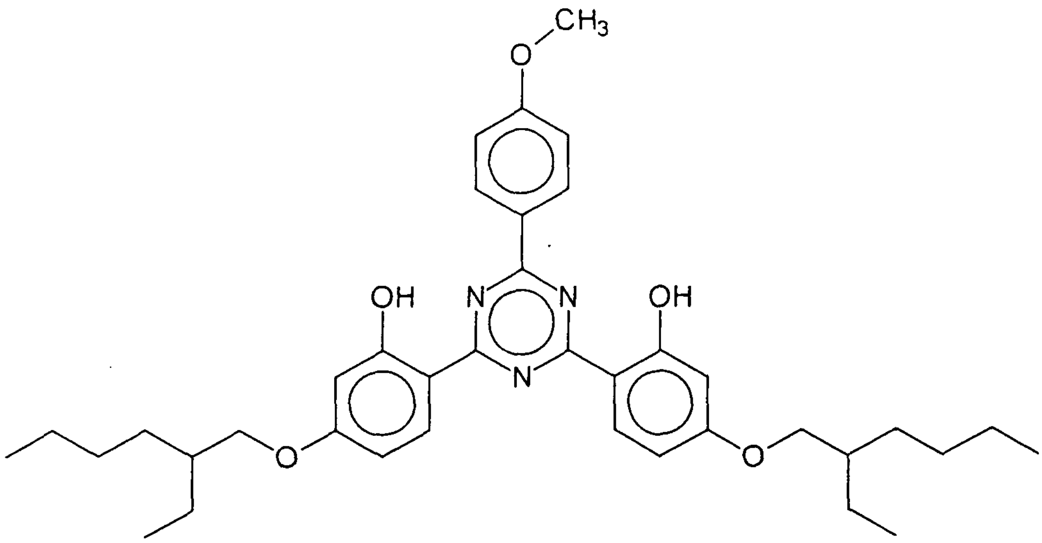

Diese UVB-Filtersubstanz wird von der Ciba Specialty Chemicals Holding Inc. unter der Warenbezeichnung Tinosorb® S vertrieben und zeichnet sich durch gute UV-Absorptionseigenschaften aus.

Der Hauptnachteil des 2,4-Bis-{[4-(2-Ethyl-hexyloxy)-2-hydroxy]-phenyl}-6-(4-methoxyphenyl)-1,3,5-triazins ist seine schlechte Löslichkeit in Lipiden. Bekannte Lösungsmittel für diesen UVB-Filter können maximal ca. 20 Gew.-% dieses Filters lösen, entsprechend etwa 2 Gew.-% gelöster, und damit aktiver, UV-Filtersubstanz. Es bereitet daher auch hier gewisse formulierungstechnische Schwierigkeiten, höhere Lichtschutzfaktoren zu erzielen.

Ein weiterer vorteilhafter UV-Filter ist das Dioctylbutamidotriazon, dessen chemische Struktur durch die Formel

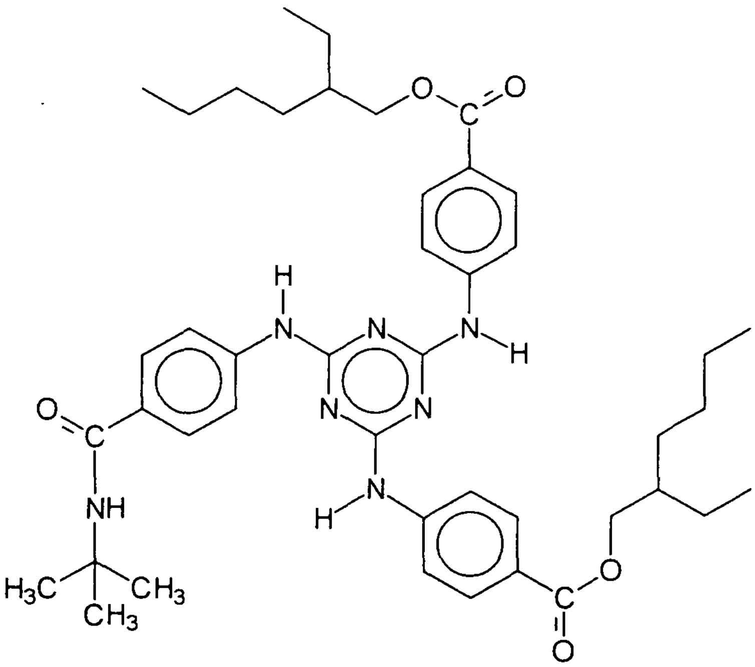

wiedergegeben wird, welche im Folgenden auch als Dioctylbutamidotriazon bezeichnet werden. Diese UV-Filtersubstanz wird von Gesellschaft 3V SIGMA unter der Warenbezeichnung Uvasorb HEB vertrieben und zeichnet sich durch gute UV-Absorptionseigenschaften aus. Ferner zeichnet sich diese Substanz durch die Eigenschaft aus, auf der Haut unmerklich dünne Filme zu bilden, die einesteils erwünscht sein können, andererseits aber auch taktile Nachteile aufweisen.

Es war also eine Aufgabe der vorliegenden Erfindung, den Nachteilen des Standes der Technik abzuhelfen. Ferner war es eine Aufgabe der vorliegenden Erfindung, kosmetische oder dermatologische Lichtschutzzubereitungen zu konzipieren, welche sich durch erhöhte Pflegewirkung und durch eine kosmetisch ansprechende Anmutung auszeichnen.

Der Lichtschutzfaktor (LSF, oft auch, dem englischen Sprachgebrauch angepaßt, SPF genannt) gibt an, um wieviel länger die mit dem Lichtschutzmittel geschützte Haut bestrahlt werden kann, bis die gleiche Erythemreaktion auftritt wie bei der ungeschützten Haut (also zehnmal solang gegenüber ungeschützter Haut bei LSF = 10).

Jedenfalls erwartet der Verbraucher zum einen - nicht zuletzt wegen der ins Licht der Öffentlichkeit gerückten Diskussion über das sogenannte "Ozonloch" zum einen zuverlässige Angaben des Herstellers zum Lichtschutzfaktor, zum anderen geht eine Tendenz des Verbrauchers zu höheren und hohen Lichtschutzfaktoren.

Da Lichtschutzfiltersubstanzen in der Regel kostspielig sind, und da manche Lichtschutzfiltersubstanzen zudem schwierig in höheren Konzentrationen in kosmetische oder dermatologische Zubereitungen einzuarbeiten sind, war es eine weitere Aufgabe der Erfindung, auf einfache und preiswerte Weise zu Zubereitungen zu gelangen, welche bei ungewöhnlich niedrigen Konzentrationen an herkömmlichen Lichtschutzfiltersubstanzen dennoch akzeptable oder sogar hohe LSF-Werte erreichen.

Es war also eine Aufgabe der vorliegenden Erfindung, den Nachteilen des Standes der Technik abzuhelfen. Ferner war es eine Aufgabe der vorliegenden Erfindung, kosmetische oder dermatologische Lichtschutzzubereitungen zu konzipieren, welche sich durch erhöhte Pflegewirkung auszeichnen.

Überraschenderweise werden diese Aufgaben gelöst durch kosmetische oder dermatologische Lichtschutzzubereitungen mit einem Gehalt an

(a) Dioctylbutamidotriazon,
(b) einem oder mehreren Filmbildnern sowie
(c) einem oder mehreren Polyolen
sowie die Verwendung von Dioctylbutamidotriazon

(a) Dioctylbutamidotriazon,
(b) einem oder mehreren Filmbildnern sowie
(c) einem oder mehreren Polyolen

zur Herstellung kosmetisch oder dermatologisch akzeptabler Zubereitungen welche einen lichtschutzwirksamen Film auf der Haut hinterlassen.

Filmbildner im Sinne der vorliegenden Erfindung sind Stoffe unterschiedlicher Zusammensetzung, die durch die folgende Eigenschaft charakterisiert sind: Löst man einen Filmbildner in Wasser oder anderen geeigneten Lösungsmitteln und trägt die Lösung dann auf die Haut auf, so bildet er nach dem Verdunsten des Lösemittels einen Film aus, der im wesentlichen eine Schutzfunktion hat.

Ein zweiter Filmbildnertyp umfaßt Substanzen, welche in einem Lösemittel, welches nicht notwendigerweise verdunsten muß, nach dem Auftragen Filme bilden. In einem solchen Falle handelt es sich zumeist um öllösliche polymere Grundkörper.

Es ist insbesondere von Vorteil, die Filmbildner aus der Gruppe der Polymere auf Basis von Polyvinylpyrrolidon (PVP)

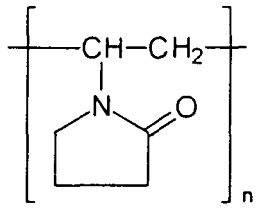

zu wählen. Besonders bevorzugt sind Copolymere des Polyvinylpyrrolidons, beispielsweise das PVP Hexadecen Copolymer und das PVP Eicosen Copolymer, welche unter den Handelsbezeichnungen Antaron V216 und Antaron V220 bei der GAF Chemicals Cooperation erhältlich sind.

Erfindungsgemäß vorteilhafte Filmbidner sind ferner Acrylat-Alkylacrylat-Copolymere, insbesondere solche, die aus der Gruppe der sogenannten Carbomere oder Carbopole (Carbopol® ist eigentlich eine eingetragene Marke der B. F. Goodrich Company) gewählt werden. Insbesondere zeichnen sich das oder die erfindungsgemäß vorteilhaften Acrylat-Alkylacrylat-Copolymere durch die folgende Struktur aus:

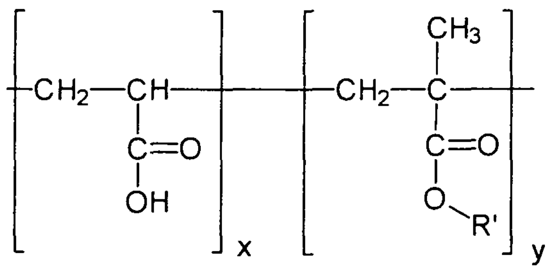

Darin stellen R' einen langkettigen Alkylrest und x und y Zahlen dar, welche den jeweiligen stöchiometrischen Anteil der jeweiligen Comonomere symbolisieren.

Erfindungsgemäß besonders bevorzugt sind Acrylat-Copolymere und/oder Acrylat-Alkylacrylat-Copolymere, welche unter den Handelbezeichnungen Carbopol® 1382, Carbopol® 981 und Carbopol® 5984 von der B. F.Goodrich Company erhältlich sind.

Ferner vorteilhaft sind Copolymere aus $C_{10-30}$-Alkylacrylaten und einem oder mehreren Monomeren der Acrylsäure, der Methacrylsäure oder deren Ester, die kreuzvernetzt sind mit einem Allylether der Saccharose oder einem Allylether des Pentaerythrit.

Vorteilhaft sind Verbindungen, die die INCI-Bezeichnung "Acrylates/C 10-30 Alkyl Acrylate Crosspolymer" tragen. Insbesondere vorteilhaft sind die unter den Handelsbezeichnungen Pemulen TR1 und Pemulen TR2 bei der B. F. Goodrich Company erhältlichen.

Die Gesamtmenge an einem oder mehreren Polyacrylaten wird in den fertigen kosmetischen oder dermatologischen Hydrodispersionen vorteilhaft kleiner als 5,0 Gew.-%, bevorzugt zwischen 0,01 und 1,0 Gew.-%, bezogen auf das Gesamtgewicht der Zubereitungen, gewählt.

Es kann im Sinne der vorliegenden Erfindung ferner von Vorteil sein, wasserlösliche und/oder in Wasser quellbare und/oder mit Hilfe von Wasser gelierbare Polysaccharide als Filbildner zu verwenden. Von besonderem Vorteil können verwendet werden Hyaluronsäure, Chitosan sowie auch andere wasserlösliche und/oder in Wasser quellbare

und/oder mit Hilfe von Wasser gelierbare Polysaccharide zu verwenden, beispielsweise ein fucosereiches Produkt, welches in den Chemical Abstracts unter der Registraturnummer 178463-23-5 abgelegtes Polysaccharid, welches unter der Bezeichnung Fucogel®1000 von der Gesellschaft SOLABIA S.A. erhältlich ist. Letzteres zeichnet sich durch Strukturelemente aus wie folgt:

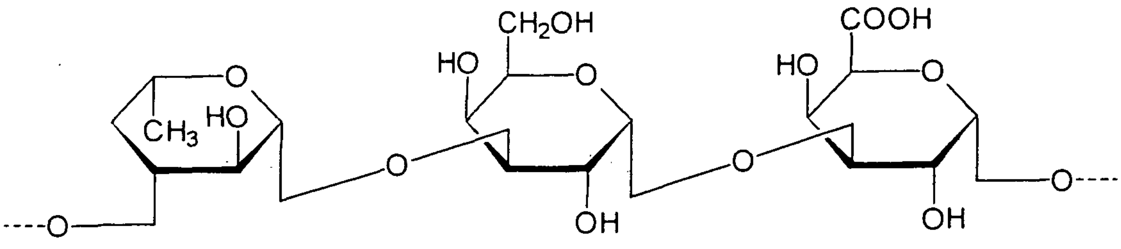

Hyaluronsäure zeichnet sich durch die Struktur

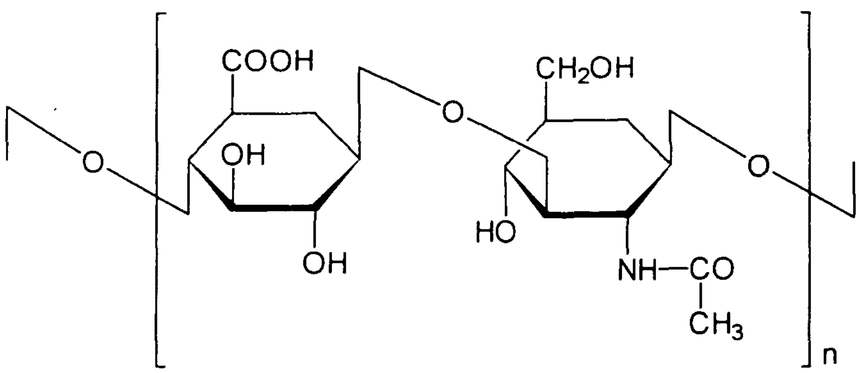

aus.

Wenn Hyaluronsäure den oder einen der verwendeten Filmbildner darstellt, ist es von Vorteil, solche mit Molekulargewichten zwischen 30.000 und 8.000.000 zu wählen, insbesondere solche mit Molekulargewichten zwischen 500.000 und 1.500.000.

Chitosan ist gekennzeichnet durch folgende Strukturformel:

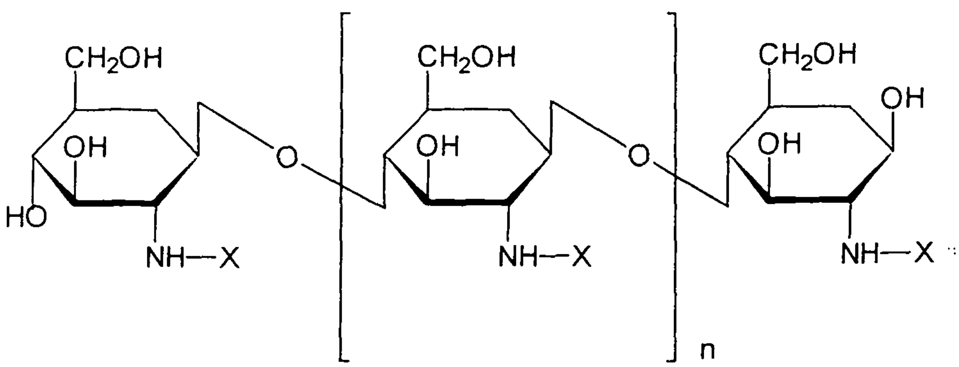

dabei nimmt n Werte bis zu ca. 2.000 an, X stellt entweder den Acetylrest oder Wasserstoff dar. Chitosan entsteht durch Deacetylierung und teilweise Depolymerisation (Hydrolyse) von Chitin, welches durch die Strukturformel

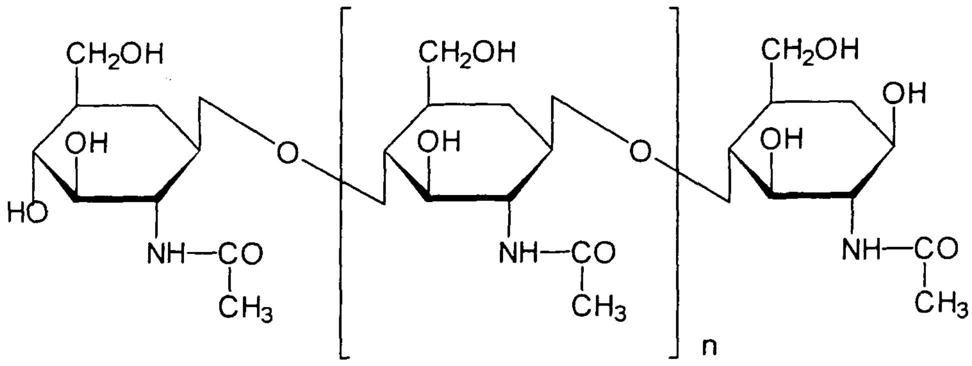

gekennzeichnet ist. Chitin ist wesentlicher Bestandteil des Ektoskeletts

['ο χιτων =

grch.: der Panzerrock]

der Gliederfüßer (z.B. Insekten, Krebse, Spinnen) und wird auch in Stützgeweben anderer Organismen (z.B. Weichtiere, Algen, Pilze) gefunden.

Chitosan ist ein in der Haarpflege bekannter Rohstoff. Es eignet sich, besser als das ihm zugrundeliegende Chitin, als Verdicker oder Stabilisator und verbessert die Adhäsion und Wasserresistenz von polymeren Filmen. Stellvertretend für eine Vielzahl von Fundstellen des Standes der Technik: H.P.Fiedler, "Lexikon der Hilfsstoffe für Pharmazie, Kosmetik und angrenzende Gebiete", dritte Auflage 1989, Editio Cantor, Aulendorf, S. 293, Stichwort "Chitosan".

Erfindungsgemäß bevorzugt sind Chitosane mit einem Deacetylierungsgrad > 60 %, insbesondere > 80%. Unter diesen besonders bevorzugt sind solche, deren 1%-ige wäßrige Lösung eine Viskosität von 4.500 - 5.500 mPas (Brookfield, Spindel 5, 10 UpM), insbesondere 5.000 mPas aufweist.

Wenn Chitosan das oder eines der verwendeten Polysaccharide darstellt, ist es von Vorteil, solches mit Molekulargewichten zwischen 3.000 und 2.000.000 zu wählen, insbesondere solches mit Molekulargewichten zwischen 10.000 und 500.000.

Erfindungsgemäß enthalten kosmetische oder dermatologische Lichtschutzzubereitungen 0,1 bis 20 Gew.-%, vorteilhaft 0,5 bis 10 Gew.-%, ganz besonders bevorzugt 1 bis 5 Gew.-% Polysaccharide.

Ebenfalls vorteilhaft sind weitere polymere Filmbildner, wie beispielsweise Natriumpolystryrolsulfonat, welches unter der Handelsbezeichnung Flexan 130 bei der National Starch and Chemical Corp. erhältlich ist, und/oder Polyisobuten, erhältlich bei Rewo unter der Handelsbezeichnung Rewopal PIB1000.

Die Gesamtmenge an einem oder mehreren Filmbildnern wird in den fertigen kosmetischen oder dermatologischen Zubereitungen vorteilhaft kleiner als 20,0 Gew.-%, bevorzugt zwischen 0,1 und 15,0 Gew.-% besonders bevorzugt zwischen 0,5 und 7,0 Gew.-%, bezogen auf das Gesamtgewicht der Zubereitungen, gewählt.

Die Gesamtmenge an Dioctylbutamidotriazon wird in den fertigen kosmetischen oder dermatologischen Zubereitungen vorteilhaft kleiner als 15,0 Gew.-%, bevorzugt zwischen 1,0 und 10,0 Gew.-% besonders bevorzugt zwischen 2,0 und 8,0 Gew.-%, bezogen auf das Gesamtgewicht der Zubereitungen, gewählt.

Die Gesamtmenge an einem oder mehreren Polyolen wird in den fertigen kosmetischen oder dermatologischen Zubereitungen vorteilhaft kleiner als 20,0 Gew.-%, bevorzugt zwischen 1,0 und 15,0 Gew.-% besonders bevorzugt zwischen 2,0 und 10,0 Gew.-%, bezogen auf das Gesamtgewicht der Zubereitungen, gewählt.

Erfindungsgemäß können die Polyole können vorteilhaft gewählt werden aus der Gruppe der mindestens bifunktionellen Alkohole. Vorteilhaft sind insbesondere die Polyole, gewählt aus der folgenden Gruppe:

Ethylenglycol, Polyethylenglycole mit Molmassen bis zu ca. 2.000, Propylenglycol-1,2, Polypropylenglycole-1,2 mit Molmassen bis zu ca. 2.000, Propylenglycol-1,3, Polypropylenglycole-1,3 mit Molmassen bis zu ca. 2.000, Butylenglycol-1,2, Polybutylenglycole-1,2 mit Molmassen bis zu ca. 2.000, Butylenglycol-1,3, Polybutylenglycole-1,3 mit Molmassen bis zu ca. 2.000, Butylenglycol-1,4, Polybutylenglycole-1,4 mit Molmassen bis zu ca. 2.000, Butylenglycol-2,3, Polybutylenglycole-2,3 mit Molmassen bis zu ca. 2.000, Glycerin, Diglycerin, Triglycerin, Tetraglycerin und Pentaglycerin, wobei die Oligoglycerine aus über eine oder mehrere Etherbrücken kondensierten Glycerineinheiten zusammengesetzt sind, beispielsweise wie folgt:

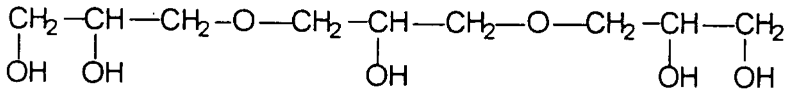

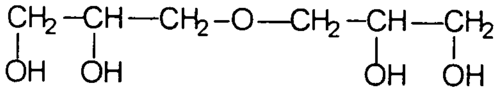

Bevorzugte Polyole sind die monomeren Butylenglycole. Ganz besonders bevorzugt sind Glycerin, 1,2-Propylenglycol und 1,3-Butylenglycol.

Es ist von Vorteil, Gewichtsverhältnisse von Dioctylbutamidotriazon und Polyolen aus dem Bereich von 1 : 10 bis 10 : 1, bevorzugt 1 : 4 bis 4 : 1, zu wählen.

Es ist ferner von Vorteil, Gewichtsverhältnisse von Dioctylbutamidotriazon und Filmbildnern aus dem Bereich von 1 : 10 bis 10 : 1, bevorzugt 1 : 4 bis 4 : 1, zu wählen.

Schließlich ist von Vorteil Gewichtsverhältnisse von Polyolen und Filmbildnern aus dem Bereich von 1 : 10 bis 10 : 1, bevorzugt 1 : 4 bis 4 : 1, zu wählen.

Die erfindungsgemäßen kosmetischen und/oder dermatologischen Lichtschutzformulierungen können wie üblich zusammengesetzt sein und dem kosmetischen und/oder dermatologischen Lichtschutz, ferner zur Behandlung, der Pflege und der Reinigung der Haut und/oder der Haare und als Schminkprodukt in der dekorativen Kosmetik dienen.

Zur Anwendung werden die erfindungsgemäßen kosmetischen und dermatologischen Zubereitungen in der für Kosmetika üblichen Weise auf die Haut und/oder die Haare in ausreichender Menge aufgebracht.

Besonders bevorzugt sind solche kosmetischen und dermatologischen Zubereitungen, die in der Form eines Sonnenschutzmittels vorliegen. Vorteilhaft können diese zusätzlich mindestens einen weiteren UVA-Filter und/oder mindestens einen weiteren UVB-Filter und/oder mindestens ein anorganisches Pigment, bevorzugt ein anorganisches Mikropigment, enthalten.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Ein zusätzlicher Gehalt an Antioxidantien ist im allgemeinen bevorzugt. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. $\alpha$-Carotin, $\beta$-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, $\gamma$-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis $\mu$mol/kg), ferner (Metall)-Chelatoren (z.B. $\alpha$-Hydroxyfettsäuren, Palmitinsäure, Phytinsäure, Lactoferrin), $\alpha$-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. $\gamma$-Linolensäure, Linolsäure, Ölsäure), Folsäure und deren Derivate, Furfurylidensorbitol und dessen Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, $\alpha$-Glycosylrutin, Ferulasäure, Furfurylidenglucitol, Camosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, $ZnSO_4$) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 0,1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Die Lipidphase kann vorteilhaft gewählt werden aus folgender Substanzgruppe:

- Mineralöle, Mineralwachse
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkemöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, $C_{12-15}$-Alkylbenzoat, Capryl-Caprinsäure-triglycerid, Dicaprylylether.

Besonders vorteilhaft sind Mischungen aus $C_{12-15}$-Alkybenzoat und 2-Ethylhexylisostearat, Mischungen aus $C_{12-15}$-Alkybenzoat und Isotridecylisononanoat sowie Mischungen aus $C_{12-15}$-Alkybenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wäßrige Phase der erfindungsgemäßen Zubereitungen enthält gegebenenfalls vorteilhaft

- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder - monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Die kosmetischen oder dermatologischen Lichtschutzzubereitungen enthalten vorteilhaft anorganische Pigmente, insbesondere Mikropigmente, z.B. in Mengen von 0,1 Gew.-% bis 30 Gew.-%, vorzugsweise in Mengen von 0,5 Gew.-% bis 10 Gew.-%, insbesondere aber 1 Gew.-% bis 6 Gew.-%, bezogen auf das Gesamtgewicht der Zubereitungen.

Kosmetische und dermatologische Formulierungen im Sinne der Erfindung enthalten vorteilhaft einen oder mehrere übliche UV-A-Filter als Einzelsubstanzen oder in beliebigen Gemischen untereinander, in der Lipidphase und/ oder in der wäßrigen Phase.

Die Gesamtmenge an UV-A-Filtersubstanzen in den fertigen kosmetischen oder dermatologischen Zubereitungen wird vorteilhaft aus dem Bereich von 0,1 bis 30 Gew.-%, bevorzugt 0,1 bis 10,0 Gew.-%, insbesondere 0,5 bis 5,0 Gew.-% gewählt, bezogen auf das Gesamtgewicht der Zubereitungen.

Erfindungsgemäße kosmetische und dermatologische Zubereitungen enthalten vorteilhaft, wenngleich nicht zwingend, weitere anorganische Pigmente, welche röntgenamorph oder nichtröntgenamorph sein können.

Röntgenamorphe Oxidpigmente sind Metalloxide oder Halbmetalloxide, welche bei Röntgenbeugungsexperimenten keine oder keine erkenntliche Kristallstruktur erkennen lassen. Oftmals sind solche Pigmente durch Flammenreaktion erhältlich, beispielsweise dadurch, daß ein Metall- oder Halbmetallhalogenid mit Wasserstoff und Luft (oder reinem Sauerstoff) in einer Flamme umgesetzt wird.

In kosmetischen, dermatologischen oder pharmazeutischen Formulierungen gemäß der Erfindung werden röntgenamorphe Oxidpigmente vorteilhaft als Verdickungs- und Thixotropierungsmittel, Fließhilfsmittel, zur Emulsions- und Dispersionsstabilisierung und als Trägersubstanz (beispielsweise zur Volumenerhöhung von feinteiligen Pulvern oder Pudern) eingesetzt, sie können aber auch der Verstärkung der Lichtschutzleistung dienen.

Bekannte und vorteilhaft in Zubereitungen gemäß der Erfindung zu verwendende röntgenamorphe Oxidpigmente sind die Siliciumoxide des Typs Aerosil® (CAS-Nr. 7631-86-9. Aerosile®, erhältlich von der Gesellschaft DEGUSSA, zeichnen sich durch geringe Partikelgröße (z.B. zwischen 5 und 40 nm) aus, wobei die Partikel als kugelförmige Teilchen sehr einheitlicher Abmessung anzusehen sind. Makroskopisch sind Aerosile® als lockere, weiße Pulver erkenntlich. Im Sinne der vorliegenden Erfindung sind röntgenamorphe Siliciumdioxidpigmente besonders vorteilhaft, und unter diesen gerade solche des Aerosil®-Typs bevorzugt.

Vorteilhafte Aerosil®-Typen sind beispielsweise Aerosil® OX50, Aerosil® 130, Aerosil® 150, Aerosil® 200, Aerosil® 300, Aerosil® 380, Aerosil® MOX 80, Aerosil® MOX 170, Aerosil® COK 84, Aerosil® R 202, Aerosil® R 805, Aerosil® R 812, Aerosil® R 972, Aerosil® R 974, Aerosil® R976.

Erfindungsgemäß vorteilhaft, wenngleich nicht zwingend, enthalten kosmetische oder dermatologische Lichtschutzzubereitungen 0,1 bis 20 Gew.-%, bevorzugt 0,5 bis 10 Gew.-%, ganz besonders bevorzugt 1 bis 5 Gew.-% röntgenamorphe Oxidpigmente.

Ferner vorteilhaft können Zubereitungen gemäß der Erfindung anorganische Pigmente, insbesondere Mikropigmente enthalten, welche nichtröntgenamorph sind, auf Basis von Metalloxiden und/oder anderen in Wasser schwerlöslichen oder unlöslichen Metallverbindungen, insbesondere der Oxide des Titans ($TiO_2$), Zinks (ZnO), Eisens (z.B. $Fe_2O_3$, Zirkoniums ($ZrO_2$), Siliciums ($SiO_2$), Mangans (z.B. MnO), Aluminiums ($Al_2O_3$), Cers (z.B. $Ce_2O_3$), Mischoxiden der entsprechenden Metalle sowie Abmischungen aus solchen Oxiden. Besonders bevorzugt handelt es sich um Pigmente auf der Basis von $TiO_2$.

Derlei Pigmente schirmen UV-Strahlung, auch UVA-Strahlung ab und sind im Sinne der Erfindung als UV-Filtersubstanzen anzusehen.

Die nichtröntgenamorphen anorganischen Pigmente liegen erfindungsgemäß vorteilhaft in hydrophober Form vor, d.h., daß sie oberflächlich wasserabweisend behandelt sind. Diese Oberflächenbehandlung kann darin bestehen, daß die Pigmente nach an sich bekannten Verfahren mit einer dünnen hydrophoben Schicht versehen werden.

Eines solcher Verfahren besteht beispielsweise darin, daß die hydrophobe Oberflächenschicht nach einer Reaktion gemäß

$$n\ TiO_2 + m\ (RO)_3Si\text{-}R' \text{ -> } n\ TiO_2\ (\text{oberfl.})$$

erzeugt wird. n und m sind dabei nach Belieben einzusetzende stöchiometrische Parameter, R und R' die gewünschten organischen Reste. Beispielsweise in Analogie zu DE-OS 33 14 742 dargestellte hydrophobisierte Pigmente sind von Vorteil.

Vorteilhafte $TiO_2$-Pigmente sind beispielsweise unter den Handelsbezeichnungen T 805 von der Firma Degussa

erhältlich.

Die Gesamtmenge an anorganischen Pigmenten, insbesondere hydrophoben anorganischen Mikropigmenten in den fertigen kosmetischen oder dermatologischen Zubereitungen wird vorteilhaft aus dem Bereich von 0,1 - 30 Gew.-%, bevorzugt 0,1 - 10,0, insbesondere 0,5 - 6,0 Gew.-% gewählt, bezogen auf das Gesamtgewicht der Zubereitungen.

Die erfindungsgemäßen kosmetischen und/oder dermatologischen Lichtschutzformulierungen können wie üblich zusammengesetzt sein und dem kosmetischen und/oder dermatologischen Lichtschutz, ferner zur Behandlung, der Pflege und der Reinigung der Haut und/oder der Haare und als Schminkprodukt in der dekorativen Kosmetik dienen.

Zur Anwendung werden die erfindungsgemäßen kosmetischen und dermatologischen Zubereitungen in der für Kosmetika üblichen Weise auf die Haut und/oder die Haare in ausreichender Menge aufgebracht.

Besonders bevorzugt sind solche kosmetischen und dermatologischen Zubereitungen, die in der Form eines Sonnenschutzmittels vorliegen. Vorteilhaft können diese zusätzlich mindestens einen weiteren UVA-Filter und/oder mindestens einen weiteren UVB-Filter und/oder mindestens ein anorganisches Pigment, bevorzugt ein anorganisches Mikropigment, enthalten.

Solche UVB-Filter können öllöslich oder wasserlöslich sein. Vorteilhafte öllösliche UVB-Filtersubstanzen sind z.B.:

- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- 2,4,6-Trianilino-(p-carbo-2'-ethyl-1'-hexyloxy)-1,3,5-triazin;
- Ester der Benzalmalonsäure, vorzugsweise 4-Methoxybenzalmalonsäuredi(2-ethylhexyl)ester;
- Ester der Zimtsäure, vorzugsweise 4-Methoxyzimtsäure(2-ethylhexyl)ester, 4-Methoxyzimtsäureisopentylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;

Vorteilhafte wasserlösliche UVB-Filtersubstanzen sind z.B.:

- Salze der 2-Phenylbenzimidazol-5-sulfonsäure wie ihr Natrium-, Kalium- oder ihr Triethanolammonium-Salz, sowie die Sulfonsäure selbst;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und deren Salze.

Die Liste der genannten UVB-Filter, die zusätzlich im Sinne der vorliegenden Erfindung eingesetzt werden können, soll selbstverständlich nicht limitierend sein.

Vorteilhaft sind auch Zubereitungen gemäß der Erfindung, dadurch gekennzeichnet, daß die kosmetischen oder dermatologischen Lichtschutzzubereitungen als UV-A-Filtersubstanz ein oder mehrere wasserlösliche UV-A-Filtersubstanzen, besonders wasserlösliche UV-A-Filtersubstanzen gewählt aus der Gruppe Phenylen-1,4-bis-(2-benzimidazyl)-3,3'-5,5'-tetrasulfonsäure und/oder 1,4-di(2-oxo-10-Sulfo-3-bornylidenmethyl)-Benzol und/oder ihre Salze, besonders die entsprechenden Natrium-, Kalium-oder Triethanolammonium-Salze und/oder die entprechenden 10-Sulfatoverbindungen, insbesondere das Phenylen-1,4-bis-(2-benzimidazyl)-3,3'-5,5'-tetrasulfonsäure-bis-natriumsalz, enthalten.

Es kann auch von erheblichem Vorteil sein, polymergebundene oder polymere UV-Filtersubstanzen in Zubereitungen gemäß der vorliegenden Erfindung zu verwenden, insbesondere solche, wie sie in der WO-A-92/20690 beschrieben werden.

Vorteilhaft sind daher auch Zubereitungen gemäß der Erfindung, enthaltend polymergebundene oder polymere UV-Filtersubstanzen, wobei die Gesamtmenge solcher Filtersubstanzen z.B. 0,1 Gew.-% bis 30 Gew.-%, vorzugsweise 0,5 bis 20 Gew.-%, insbesondere 3 bis 15 Gew.-% beträgt, bezogen auf das Gesamtgewicht der Zubereitungen, erheblich gesteigert.

Die Gesamtmenge an wasserlöslichen UV-Filtersubstanzen in den fertigen kosmetischen oder dermatologischen Zubereitungen gemäß der Erfindung wird vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an öllöslichen UV-Filtersubstanzen in den fertigen kosmetischen oder dermatologischen Zubereitungen gemäß der Erfindung wird vorteilhaft aus dem Bereich von 0,1 - 20,0 Gew.-%, bevorzugt 0,5 - 10,0 Gew.-% gewählt, bezogen auf das Gesamtgewicht der Zubereitungen.

Die Gesamtmenge an 2-Phenylbenzimidazol-5-sulfonsäure bzw. deren Salzen in den fertigen kosmetischen oder dermatologischen Zubereitungen gemäß der Erfindung wird, falls die Gegenwart dieser Substanz erwünscht ist, vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an 2-Hydroxy-4-methoxybenzophenon-5-sulfonsäure bzw. deren Salzen in den fertigen kosmetischen oder dermatologischen Zubereitungen gemäß der Erfindung wird, falls die Gegenwart dieser Substanz erwünscht ist, vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an 4-(2-Oxo-3-bornylidenmethyl)benzolsulfonsäure bzw. deren Salzen in den fertigen kosmetischen oder dermatologischen Zubereitungen gemäß der Erfindung wird, falls die Gegenwart dieser Substanz erwünscht ist, vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an 2-Methyl-5-(2-oxo-3-bornylidenmethyl)benzolsulfonsäure bzw. deren Salzen in den fertigen kosmetischen oder dermatologischen Zubereitungen gemäß der Erfindung wird, falls die Gegenwart dieser Substanz erwünscht ist, vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an Benzol-1,4-di(2-oxo-3-bornylidenmethyl-10-Sulfonsäure bzw. deren Salzen in den fertigen kosmetischen oder dermatologischen Zubereitungen gemäß der Erfindung wird, falls die Gegenwart dieser Substanz erwünscht ist, vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an 4,4',4"-(1,3,5-Triazin-2,4,6-triyltriimino)-tris-benzoesäure-tris(2-ethylhexylester) in den fertigen kosmetischen oder dermatologischen Zubereitungen gemäß der Erfindung wird, falls die Gegenwart dieser Substanz erwünscht ist, vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an 4-(tert.-Butyl)-4'-methoxydibenzoylmethan in den fertigen kosmetischen oder dermatologischen Zubereitungen gemäß der Erfindung wird, falls die Gegenwart dieser Substanz erwünscht ist, vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an 4-Methylbenzylidencampher in den fertigen kosmetischen oder dermatologischen Zubereitungen gemäß der Erfindung wird, falls die Gegenwart dieser Substanz erwünscht ist, vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an 2-Ethylhexyl-p-methoxy-cinnamat in den fertigen kosmetischen oder dermatologischen Zubereitungen gemäß der Erfindung wird, falls die Gegenwart dieser Substanz erwünscht ist, vorteilhaft aus dem Bereich von 0,1 - 15,0 Gew.-%, bevorzugt 0,5 - 7,5 Gew.-% gewählt, bezogen auf das Gesamtgewicht der Zubereitungen.

Ferner kann es gegebenenfalls von Vorteil sein, weitere UVA- und/oder UVB-Filter in kosmetische oder dermatologische Zubereitungen gemäß der Erfindung einzuarbeiten, beispielsweise bestimmten Salicylsäurederivaten wie 4-Isopropylbenzylsalicylat, 2-Ethylhexylsalicylat (=Octylsalicylat), Homomenthylsalicylat.

Die Gesamtmenge an einem oder mehreren Salicylsäurederivaten in den fertigen kosmetischen oder dermatologischen Zubereitungen gemäß der Erfindung wird vorteilhaft aus dem Bereich von 0,1 - 15,0 Gew.-%, bevorzugt 0,5 - 8,0 Gew.-% gewählt, bezogen auf das Gesamtgewicht der Zubereitungen. Wenn Ethylhexylsalicylat gewählt wird, ist es von Vorteil, dessen Gesamtmenge aus dem Bereich von 0,1 - 5,0 Gew.-%, bevorzugt 0,5 - 2,5 Gew.-% zu wählen. Wenn Homomenthylsalicylat gewählt wird, ist es von Vorteil, dessen Gesamtmenge aus dem Bereich von 0,1 - 10,0 Gew.-%, bevorzugt 0,5 - 5,0 Gew.-% zu wählen.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

Beispiel 1

| O/W - Emulsion | Gew.-% |
|---|---|
| Stearinsäure | 1,50 |
| Glycerinmonostearat | 3,00 |
| Caprylsäure/Caprinsäuretriglycerid | 10,00 |
| Dicaprylylether | 5,00 |
| Dimethicon | 2,00 |
| Hydriertes Polyisobuten | 2,00 |
| Vitamin-E-Acetat | 0,50 |
| Dioctylbutamidotriazon | 2,00 |

Tabelle fortgesetzt

| O/W - Emulsion | Gew.-% |
|---|---|
| PVP Hexadecen Copolymer | 2,00 |
| Octyltriazon | 1,00 |
| Methylbenzylidencampher | 4,00 |
| Butylmethoxydibenzoylmethan | 2,00 |
| Titandioxid | 1,00 |
| Konservierung | 0,50 |
| Butylenglycol-1,3 | 2,00 |
| Glycerin | 3,00 |
| Xanthan Gummi | 0,30 |
| Natronlauge 45% | 0,50 |
| Wasser | ad 100,00 |

Beispiel 2

| O/W - Emulsion | Gew.-% |
|---|---|
| Stearinsäure | 1,50 |
| Glycerinmonostearat | 3,00 |
| Caprylsäure/Caprinsäuretriglycerid | 5,00 |
| Dicaprylylether | 5,00 |
| Dimethicon | 1,00 |
| Isohexadekan | 2,00 |
| Butylenglycoldicaprylat/Caproat | 2,00 |
| $C_{12-15}$-Alkylbenzoate | 3,00 |
| Vitamin-E-Acetat | 0,50 |
| Dioctylbutamidotriazon | 4,00 |
| PVP Hexadecen Copolymer | 6,00 |
| Konservierung | 0,50 |
| Butylenglycol-1,3 | 5,00 |
| Glycerin | 1,00 |
| Pemulen® TR1 | 0,20 |
| Phenylbenzimidazolsulfonsäure | 3,00 |
| Natronlauge 45% | 1,50 |
| Wasser | ad 100,00 |

Beispiel 3

| O/W - Emulsion | Gew.-% |
|---|---|
| Sorbitanstearat | 3,00 |
| Polyglyceryl-3-methylglucosedistearat | 1,50 |
| Octyldodecanol | 10,00 |
| Dicaprylylether | 5,00 |
| Cetylstearylisononanoat | 2,00 |
| Butylenglycoldicaprylat/Caproat | 5,00 |
| Vitamin-E-Acetat | 0,50 |
| Dioctylbutamidotriazon | 3,00 |

Tabelle fortgesetzt

| O/W - Emulsion | Gew.-% |
|---|---|
| PVP Hexadecen-Copolymer | 2,00 |
| Octyltriazon | 4,00 |
| Methylbenzylidencampher | 4,00 |
| Butylmethoxydibenzoylmethan | 3,00 |
| Konservierung | 0,50 |
| Glycerin | 10,00 |
| Xanthan Gummi | 0,20 |
| Pemulen® TR1 | 0,10 |
| Natronlauge 45% | 0,10 |
| Wasser | ad 100,00 |

Beispiel 4

| O/W - Emulsion | Gew.-% |
|---|---|
| Sorbitanstearat | 3,00 |
| Polyglyceryl-3-methylglucosedistearat | 1,50 |
| Octyldodecanol | 10,00 |
| Dimethicon | 2,00 |
| Mineralöl | 5,00 |
| Hydriertes Polyisobuten | 5,00 |
| Butylenglycoldicaprylat/Caproat | 5,00 |
| Vitamin-E-Acetat | 0,50 |
| Dioctylbutamidotriazon | 6,00 |
| PVP Hexadecen Copolymer | 2,00 |
| Konservierung | 0,50 |
| Butylenglycol-1,3 | 5,00 |
| Glycerin | 5,00 |
| Xanthan Gummi | 0,20 |
| Pemulen® TR1 | 0,10 |
| Natronlauge 45% | 0,10 |
| Wasser | ad 100,00 |

Beispiel 5

| W/O - Emulsion | Gew.-% |
|---|---|
| Polyglyceryl-2-dipolyhydroxystearat | 5,00 |
| Dimethicon | 2,00 |
| Mineralöl | 5,00 |
| Isohexadekan | 5,00 |
| Butylenglycoldicaprylat/Caproat | 5,00 |
| $C_{12-15}$-Alkylbenzoate | 8,00 |
| Dioctylbutamidotriazon | 3,00 |
| PVP Hexadecen Copolymer | 3,00 |
| Methylbenzylidencampher | 2,00 |
| Butylmethoxydibenzoylmethan | 2,00 |

Tabelle fortgesetzt

| W/O - Emulsion | Gew.-% |
|---|---|
| Titandioxid | 4,00 |
| Konservierung | 0,50 |
| Glycerin | 5,00 |
| $MgSO_4$ | 1,00 |
| Wasser | ad 100,00 |

Beispiel 6

| W/O - Emulsion | Gew.-% |
|---|---|
| PEG-30-dipolyhydroxystearat | 4,00 |
| Caprylsäure/Caprinsäuretriglycerid | 8,00 |
| Octyldodecanol | 5,00 |
| Dicaprylylether | 5,00 |
| Dimethicon | 2,00 |
| Mineralöl | 5,00 |
| Isohexadekan | 5,00 |
| Hydriertes Polyisobuten | 5,00 |
| Vitamin-E-Acetat | 0,50 |
| Dioctylbutamidotriazon | 1,00 |
| PVP Hexadecen Copolymer | 2,00 |
| Aerosil® R 972 | 0,50 |
| Konservierung | 0,50 |
| Glycerin | 10,00 |
| NaCl | 1,00 |
| Phenylbenzimidazolsulfonsäure | 1,00 |
| Natronlauge 45% | 0,30 |
| Wasser | ad 100,00 |

Beispiel 7

| W/O - Emulsion | Gew.-% |
|---|---|
| Cetyldimethiconcopolyol | 5,00 |
| Dimethicon | 5,00 |
| Mineralöl | 2,00 |
| Isohexadekan | 2,00 |
| Butylenglycoldicaprylat/Caproat | 8,00 |
| $C_{12-15}$-Alkylbenzoate | 5,00 |
| Dioctylbutamidotriazon | 4,00 |
| PVP Hexadecen Copolymer | 4,00 |
| Methylbenzylidencampher | 4,00 |
| Butylmethoxydibenzoylmethan | 2,00 |
| Titandioxid | 2,00 |
| Konservierung | 0,50 |
| Butylenglycol-1,3 | 2,00 |
| Glycerin | 5,00 |

Tabelle fortgesetzt

| W/O - Emulsion | Gew.-% |
|---|---|
| $MgSO_4$ | 1,00 |
| Wasser | ad 100,00 |

Beispiel 8

| Spray | Gew.-% |
|---|---|
| Glycerinmonostearat | 4,00 |
| Ceteareth-12 | 1,50 |
| Caprylsäure/Caprinsäuretriglycerid | 2,00 |
| Mineralöl | 5,00 |
| Dioctylbutamidotriazon | 0,50 |
| PVP Hexadecen Copolymer | 4,00 |
| Octyltriazon | 1,00 |
| Konservierung | 0,50 |
| Glycerin | 10,00 |
| Phenylbenzimidazolsulfonsäure | 4,00 |
| Natronlauge 45% | 1,30 |
| Wasser | ad 100,00 |

Beispiel 9

| Spray | Gew.-% |
|---|---|
| Glycerinmonostearat SE | 4,50 |
| Ceteareth-20 | 1,00 |
| Dicaprylylether | 5,00 |
| Cetylstearylisononanoat | 5,00 |
| Dimethicon | 2,00 |
| Dioctylbutamidotriazon | 3,00 |
| PVP Hexadecen Copolymer | 2,00 |
| Methylbenzylidencampher | 2,00 |
| Butylmethoxydibenzoylmethan | 1,00 |
| Konservierung | 0,50 |
| Glycerin | 5,00 |
| Wasser | ad 100,00 |

**Patentansprüche**

**1.** Kosmetische oder dermatologische Lichtschutzzubereitungen mit einem Gehalt an

(a) Dioctylbutamidotriazon,
(b) einem oder mehreren Filmbildnern sowie
(c) einem oder mehreren Polyolen.

**2.** Lichtschutzzubereitungen nach Anspruch 1, **dadurch gekennzeichnet, daß** der oder die Filmbildner aus der Gruppe der Polymere auf Basis von Polyvinylpyrrolidon, der Copolymere des Polyvinylpyrrolidons, beispielsweise das PVP

Hexadecen Copolymer und das PVP Eicosen Copolymer, der Acrylat-Polymere, der Acrylat-Alkylacrylat-Copolymere, insbesondere solche, die aus der Gruppe der sogenannten Carbomere oder Carbopole der Copolymere aus $C_{10-30}$-Alkylacrylaten und einem oder mehreren Monomeren der Acrylsäure, der Methacrylsäure oder deren Ester, die kreuzvemetzt sind mit einem Allylether der Saccharose oder einem Allylether des Pentaerythrit, der Polysaccharide, beispielsweise Hyaluronsäure oder Chitosan gewählt werden.

**3.** Lichtschutzzubereitungen nach Anspruch 1, **dadurch gekennzeichnet, daß** das oder die Polyole gewählt werden aus der Gruppe Ethylenglycol, Polyethylenglycole mit Molmassen bis zu ca. 2.000, Propylenglycol-1,2, Polypropylenglycole-1,2 mit Molmassen bis zu ca. 2.000, Propylenglycol-1,3, Polypropylenglycole-1,3 mit Molmassen bis zu ca. 2.000, Butylenglycol-1,2, Polybutylenglycole-1,2 mit Molmassen bis zu ca. 2.000, Butylenglycol-1,3, Polybutylenglycole-1,3 mit Molmassen bis zu ca. 2.000, Butylenglycol-1,4, Polybutylenglycole-1,4 mit Molmassen bis zu ca. 2.000, Butylenglycol-2,3, Polybutylenglycole-2,3 mit Molmassen bis zu ca. 2.000, Glycerin, Diglycerin, Triglycerin, Tetraglycerin und Pentaglycerin.

**4.** Lichtschutzzubereitungen nach Anspruch 1, **dadurch gekennzeichnet, daß** die Gesamtmenge an einem oder mehreren Polyolen in den fertigen kosmetischen oder dermatologischen Zubereitungen vorteilhaft kleiner als 20,0 Gew.-%, bevorzugt zwischen 1,0 und 15,0 Gew.-% besonders bevorzugt zwischen 2,0 und 10,0 Gew.-%, bezogen auf das Gesamtgewicht der Zubereitungen, gewählt wird.

**5.** Lichtschutzzubereitungen nach Anspruch 1, **dadurch gekennzeichnet, daß** sie Dioctylbutamidotriazon in einer Konzentration kleiner als 15,0 Gew.-%, bevorzugt zwischen 1,0 und 10,0 Gew.-% besonders bevorzugt zwischen 2,0 und 8,0 Gew.-%, bezogen auf das Gesamtgewicht der Zubereitungen, enthalten.

**6.** Lichtschutzzubereitungen nach Anspruch 1, **dadurch gekennzeichnet, daß** die Gesamtkonzentration an Filmbildnern kleiner als 20,0 Gew.-%, bevorzugt zwischen 0,1 und 15,0 Gew.-% besonders bevorzugt zwischen 0,5 und 7,0 Gew.-%, bezogen auf das Gesamtgewicht der Zubereitunge gewählt wird.

**7.** Lichtschutzzubereitungen nach Anspruch 1, **dadurch gekennzeichnet, daß** Gewichtsverhältnisse von Dioctylbutamidotriazon und Polyolen aus dem Bereich von 1 : 10 bis 10 : 1, bevorzugt 1 : 4 bis 4 : 1, gewählt werden.

**8.** Lichtschutzzubereitungen nach Anspruch 1, **dadurch gekennzeichnet, daß** Gewichtsverhältnisse von Dioctylbutamidotriazon und Filmbildnern aus dem Bereich von 1 : 10 bis 10 : 1, bevorzugt 1 : 4 bis 4 : 1, gewählt werden.

**9.** Lichtschutzzubereitungen nach Anspruch 1, **dadurch gekennzeichnet, daß** Gewichtsverhältnisse von Polyolen und Filmbildnern aus dem Bereich von 1 : 10 bis 10 : 1, bevorzugt 1 : 4 bis 4 : 1, gewählt werden.

Europäisches Patentamt

# EUROPÄISCHER RECHERCHENBERICHT

Nummer der Anmeldung
EP 05 01 1451

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| E | WO 00/66075 A (COGNIS DEUTSCHLAND GMBH)<br>9. November 2000 (2000-11-09)<br>* Seite 11, letzter Absatz *<br>* Beispiel 1; Tabelle 1 *<br>* Beispiele 1-22; Tabelle 2 *<br>* Beispiele 27,40; Tabelle 3 *<br>* Ansprüche *<br>----- | 1-10 | A61K7/42 |
| P,X | WO 99/53895 A (BEIERSDORF AG)<br>28. Oktober 1999 (1999-10-28)<br>* Seite 14, Absatz 3 *<br>* Seite 17, Zeilen 5,6, Absatz 3 *<br>* Beispiele 1-5 *<br>* Ansprüche 1,3,4 *<br>----- | 1-10 | |
| P,X | EP 0 998 910 A (BEIERSDORF AG)<br>10. Mai 2000 (2000-05-10)<br>* Beispiel 1 *<br>----- | 1,3-9 | |
| P,X | EP 1 000 611 A (BEIERSDORF AG)<br>17. Mai 2000 (2000-05-17)<br>* Beispiel 1 *<br>----- | 1-10 | RECHERCHIERTE SACHGEBIETE (Int.Cl.7)<br>A61K |
| Y | EP 0 850 935 A (3V SIGMA SPA)<br>1. Juli 1998 (1998-07-01)<br>* Seite 3, Zeile 6 - Zeile 33 *<br>* Seite 4, Zeile 22 *<br>* Seite 4, Zeile 24 - Zeile 26 *<br>----- | 1-10 | |
| Y | US 5 656 263 A (FRUCTUS ALAIN)<br>12. August 1997 (1997-08-12)<br>* das ganze Dokument *<br>----- | 1-10 | |

Der vorliegende Recherchenbericht wurde für alle Patentansprüche erstellt

| Recherchenort | Abschlußdatum der Recherche | Prüfer |
|---|---|---|
| München | 25. August 2005 | Vayssié, S |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
.......................................................
& : Mitglied der gleichen Patentfamilie, übereinstimmendes Dokument

1
EPO FORM 1503 03.82 (P04C03)

## ANHANG ZUM EUROPÄISCHEN RECHERCHENBERICHT ÜBER DIE EUROPÄISCHE PATENTANMELDUNG NR. EP 05 01 1451

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

25-08-2005

| Im Recherchenbericht angeführtes Patentdokument | Datum der Veröffentlichung | Mitglied(er) der Patentfamilie | Datum der Veröffentlichung |
|---|---|---|---|
| WO 0066075 A | 09-11-2000 | DE 19919630 A1 | 16-11-2000 |
| | | AU 4750700 A | 17-11-2000 |
| | | WO 0066075 A1 | 09-11-2000 |
| WO 9953895 A | 28-10-1999 | DE 19817295 A1 | 21-10-1999 |
| | | DE 59909961 D1 | 19-08-2004 |
| | | WO 9953895 A1 | 28-10-1999 |
| | | EP 1071394 A1 | 31-01-2001 |
| | | ES 2224653 T3 | 01-03-2005 |
| | | JP 2002512179 T | 23-04-2002 |
| | | US 6372199 B1 | 16-04-2002 |
| EP 0998910 A | 10-05-2000 | DE 19846772 A1 | 13-04-2000 |
| | | AT 269052 T | 15-07-2004 |
| | | DE 59909736 D1 | 22-07-2004 |
| | | EP 0998910 A1 | 10-05-2000 |
| | | ES 2221727 T3 | 01-01-2005 |
| EP 1000611 A | 17-05-2000 | DE 19846771 A1 | 13-04-2000 |
| | | EP 1000611 A1 | 17-05-2000 |
| EP 0850935 A | 01-07-1998 | IT MI962725 A1 | 23-06-1998 |
| | | EP 0850935 A2 | 01-07-1998 |
| | | JP 10310514 A | 24-11-1998 |
| US 5656263 A | 12-08-1997 | FR 2702391 A1 | 16-09-1994 |
| | | US 5958435 A | 28-09-1999 |
| | | AT 172109 T | 15-10-1998 |
| | | CA 2118761 A1 | 12-09-1994 |
| | | DE 69413867 D1 | 19-11-1998 |
| | | DE 69413867 T2 | 08-04-1999 |
| | | DK 614660 T3 | 23-06-1999 |
| | | EP 0614660 A1 | 14-09-1994 |
| | | ES 2122181 T3 | 16-12-1998 |
| | | IL 108738 A | 29-02-2000 |
| | | US 5576064 A | 19-11-1996 |

EPO FORM P0461

Für nähere Einzelheiten zu diesem Anhang : siehe Amtsblatt des Europäischen Patentamts, Nr.12/82